Europäisches Patentamt

(19) European Patent Office

Office européen des brevets

(11) Publication number: **0 190 921**
A2

(12) # EUROPEAN PATENT APPLICATION

(21) Application number: 86300748.0

(22) Date of filing: 04.02.86

(51) Int. Cl.⁴: **C 12 N 15/00**
**C 12 P 13/04, C 12 P 13/22**

(30) Priority: 04.02.85 US 698099

(43) Date of publication of application:
13.08.86 Bulletin 86/33

(84) Designated Contracting States:
AT BE CH DE FR GB IT LI LU NL SE

(71) Applicant: ENGENICS, INC.
3760 Haven Avenue
Menlo Park California 94025(US)

(72) Inventor: Talkington, Carol
58 El Camino Real North, No. 318
San Mateo California 94401(US)

(74) Representative: Bizley, Richard Edward et al,
BOULT, WADE & TENNANT 27 Furnival Street
London EC4A 1PQ(GB)

(54) Method for the overproduction of amino acids.

㊺ A method is provided for overexpressing an amino acid in a microorganism comprising the step of altering the chromosomal DNA of host microorganisms or transforming the host microorganisms with at least one expression vector such that the resultant microorganism will overexpress certain operons and be inactive with respect to the other operons in the biosynthetic pathway of the amino acid, whereby overexpression of the amino acid will occur.

EP 0 190 921 A2

- 1 -

# METHOD FOR THE OVERPRODUCTION OF AMINO ACIDS

The present invention is directed to methods for overproducing one or more amino acids in a microorganism. In particular, the present invention is directed to methods for altering the genomic DNA of the host strain and/or transforming selected microorganisms with vectors in a manner such that the desired amino acids are overexpressed in the microorganism.

Substantially pure L-amino acids may be obtained from protein sources by hydrolysis, such as from whey and various fermentation mixtures. However, to obtain specific amino acids, the more typical procedure is to utilize a microorganism in fermentation which ferments a carbon source, such as glucose, to a high concentration of a single amino acid. Such microorganisms which overproduce a single amino acid are known and include species of Arthrobacter, Bacillus, Candida, Brevibacterium, Corynebacterium, Microbacterium, Micrococcus, Staphylococcus, and Streptomyces. In order to cause these organisms to overproduce a given amino acid, random mutagenesis is carried out followed by laborious methods of selection screening. The genetics of most of these strains are not sufficiently understood to allow for directed strain construction.

There are reported some organisms which fortuitously produce two amino acids, specifically phenylalanine and tryptophan (Shiio, et al., Agr. Biol. Chem. 37, 1991 (1973)) and lysine and threonine (U.S. Patent No. 3,732,144). However, the levels of production of the pairs of amino acids in the respective microorganisms are too low to be economically useful.

Recently, amino acid overproducing microorganisms in E. coli have been developed utilizing recombinant DNA techniques, i.e., by transforming E. coli with plasmids containing DNA fragments that allow for overproduction of lysine, threonine, tryptophan, histidine, valine, glutamate, phenylalanine, and arginine, respectively. See, for example, U.S. Patent Nos. 4,346,170, 4,347,318, 4,371,614, 4,288,405, 4,391,907, 4,393,135, 4,407,952, and 4,430,430. However, the use of a systematic approach combining both classical genetics and molecular biology has not previously been carried out.

The invention enables the provision of methods for overproducing one or more amino acids by fermentation of a microorganism;

production of DNA vectors which facilitate the overproduction in a microorganism of one or more amino acids;

production of transformant strains of microogranisms which overproduce one or more amino acids.

In particular, the invention enables the provision of methods for overproducing phenylalanine by fermentation of an E. coli microorganism;

production of DNA vectors which facilitate the overproduction of phenylalanine in an E. coli microorganism; and

production of transformant strains of microorganisms which overproduce phenylalanine.

The present invention provides methods for overproducing one or more amino acids in a microorganism, whereby the amino acid is expressed at an economically useful level. The methods include gene amplification, deregulation of gene expression (including repression and attenuation), resistance to feedback inhibition, and inhibition of byproduct synthesis. Specifically, the method comprises altering the host strain's chromosomal DNA (genome) and/or transforming the microorganism with at least one expression vector resulting in the following:

(a) overexpression of at least one enzyme necessary for the biosynthesis of an amino acid;

(b) elimination of feedback inhibition of expression of the amino acid by mutation of at least one gene necessary for the biosynthesis of the amino acid so that expression thereof is not affected by feedback inhibition; and

(c) inactivation of at least one gene which produces a product which inhibits or represses expression of the enzyme in step (a).

(d) inactivation of genes or operons which allow for the synthesis of byproducts that would reduce phenylalanine yields. Such byproducts are tyrosine and tryptophan.

In the accompanying figures:

Fig. 1 is a schematic representation of the biochemical pathway in E. coli. by which phe, trp and tyr are produced and regulated.

Fig. 2 is the sequence of the pheA-attenuator region in E. coli.

Fig. 3 is a schematic representation of a method by which the pheA-attenuator of E. coli is inactivated.

Fig. 4 is a schematic representation of the steps for site-specific mutagenesis of the pheA-attenuator on a plasmid.

Fig. 5 is a schematic of the construction of plasmid phe14.

Fig. 6 is a schematic of the construction of plasmid tyr25.

Fig. 7 is a schematic of the construction of plasmid phearo2.

Fig. 8 is a schematic of constructions for ligating the coding region of the pheA gene to aroF.

As used herein the term "vector" means any DNA mole-cule which has been modified by man in a manner such that the nucleotide sequences in the molecule are not identical to a sequence which is naturally produced. The term vector also includes clones of DNA molecules which have been so modified. The term "expression vector" is a vector which includes an autonomous site of replication in a host organism, a site of transcription initiation and a gene encoding a protein which is to be expressed. The expression vector will usually also contain appropriate control regions such as a promoter and terminator which control the expression of the protein. Usually, expression vectors will also contain a selection marker such as a sequence which imposes upon the transformed microorganism resistance to an antibiotic.

The term "plasmid" will have its commonly accepted meaning: an autonomously replicating usually closed-circle, fragment of DNA.

The term "operon" will also have its commonly accepted meaning, i.e., a fragment of DNA which includes coding regions for more than one protein whose expression is jointly controlled by one set of elements, usually including a promoter, operator, attenuator and terminator. The promoter is a region which is recognized and bound to by RNA polymerase, thereby leading to the transcription of the gene coding region of the operon. The operator region is the DNA segment which can bind to a repressor protein, thereby blocking transcription. The attenuator is a transcription termination element located just before the start of the coding sequences. The attenuator region can exist in two configurations, a termination mode and an antitermination mode. The terminator region allows for the release of RNA polymerase, thereby terminating transcription.

The biosynthetic pathways for amino acids in bacteria, such as E. coli, are reasonably well understood. In order to practice the present invention it is necessary to identify at least the major enzymes required to produce a particular amino acid in bacteria, as well as the various regulation mechanisms which the microorganism utilizes to repress or inhibit the expression of the amino acid. In addition, it is necessary to know about enzymes produced by the host microorganism which degrade the desired amino acids. A recent review of amino acid biosynthetic pathways in bacteria, such as E. coli, is presented in Amino acids: Biosynthesis and Genetic Regulation (Herrmann, K. M. & Somerville, R. L. eds., Addison-Wesley Publishing Co., 1983).

In the preferred embodiment of the present invention, modifications are made to the biosynthetic pathways for the production of phenylalanine in E. coli by recombinant DNA techniques resulting in the overproduction of this amino acid in E. coli.

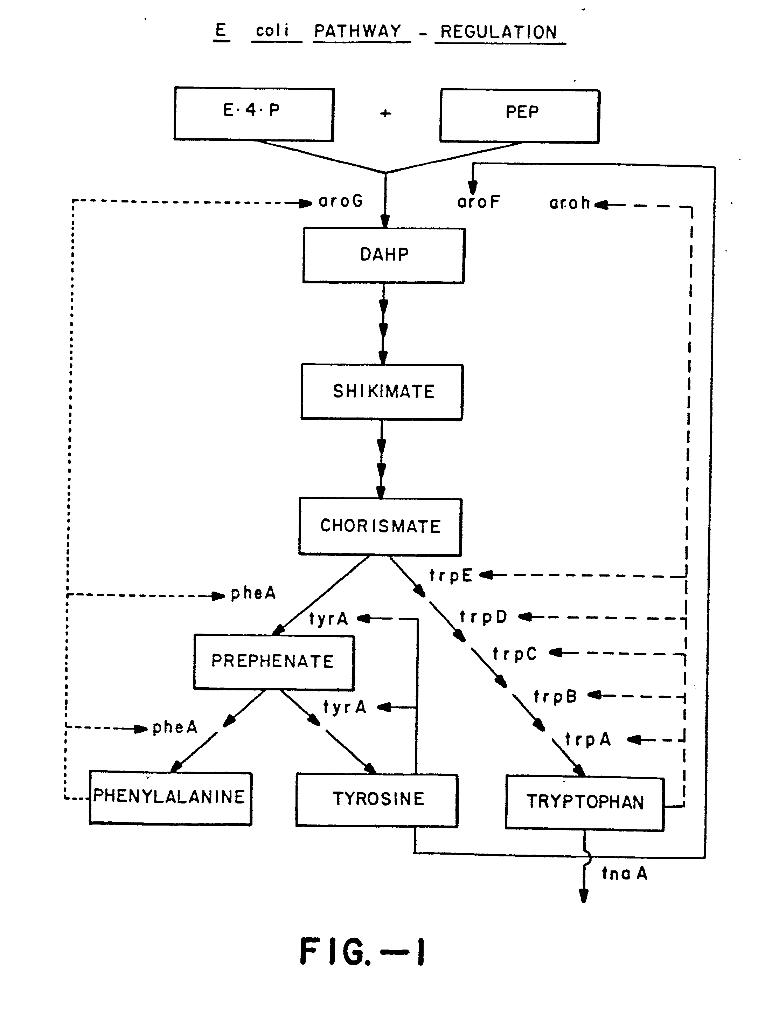

Referring to FIG. 1, there is shown the E. coli. pathway for the production of aromatic amino acids. The expression and function of two enzymes of the pathway to phenylalanine are regulated: DAHP synthetase (phospho-2-keto-3-deoxyheptonate aldolase) and chorismate mutase-prephenate dehydratase. The genes aroF, aroG, and aroH encode three isozymes of DAHP synthetase each of which condenses phosphoenol pyruvate (PEP) and erythrose-4-phosphate (E4P) into DAHP. However, the enzymes encoded by aroF, aroG, and aroH are inhibited by tyrosine, phenylalanine, and tryptophan, respectively. Excess tyrosine, phenylalanine, or tryptophan also represses expression of the aroF, aroG, or aroH gene, respectively. The gene pheA encodes the enzyme chorismate mutase-prephenate dehydratase. High concentrations of phenylalanine inhibit the function of the pheA gene product and also repress expression of the gene itself. Thus, to cause E. coli to overproduce phenylalanine, the strategy is to block the production of tyrosine by mutating tyrA (resulting in a nonfunctional protein) and to increase the expression of aroF and pheA, both by increased gene dosage, deregulation, and feedback resistance.

While the present preferred embodiment is described in connection with the biosynthetic pathway in the bacteria of E. coli, appropriate modification in the biosynthetic pathways of other bacteria may be performed according to the present invention, including species of Bacillus, Lactobacillus, Corynebacteria, and Brevibacteria.

One of the aspects according to the present invention is to overexpress at least one enzyme of the host organism necessary for the biosynthesis of the desired amino acid. One of these enzymes in the preferred embodiment may be the enzyme which shuttles carbon into the phenylalanine pathway by condensing PEP and E4P. In the case of E. coli, overexpression of the aroF gene is chosen. This overexpression is accomplished by introducing multicopy plasmid vectors containing the aroF gene into the host organism. This produces numerous autonomously replicating sources of aroF, including both that on the genomic DNA as well as those on the plasmids. Techniques for inserting a gene into a plasmid are well known. See, for example, Hershfield, et al, (1974) Proc. Natl. Acad. Sci. 71, 3455. The BamHI-BglII DNA fragment containing the aroF gene (subcloned from a plasmid disclosed by Zurawski, et al, (1978 Proc. Natl. Acad. Sci. 75, 4272) may be inserted into the appropriate restriction site (e.g., BamHI) in such well known plasmids as pBR322 and its derivatives, ColEI and its derivatives, etc. Transformation of plasmids into bacteria and other microorganisms is well known. For example, transformation of a plasmid into a bacterium may be accomplished according to the method of Hershfield, et al., op cit.

The overexpression of aroF is important since it results in expression of DAHP synthetase, an enzyme which allows for more carbon to enter the aromatic amino acid biosynthetic pathway. Also, since DAHP synthetase is fairly unstable, gene amplification will allow for continual replacement of degraded enzyme molecules.

As a modification, and to further increase expression of DAHP synthetase, an aroF gene may also be selected whose expression is constitutive due to a mutation in either the tyrR repressor or the aroF operator that disallows interaction between the repressor and operator. A feedback resistant aroF gene is selected by growth on high concentrations of tyrosine or in the presence of a tyrosine analog such as 3-fluorotyrosine.

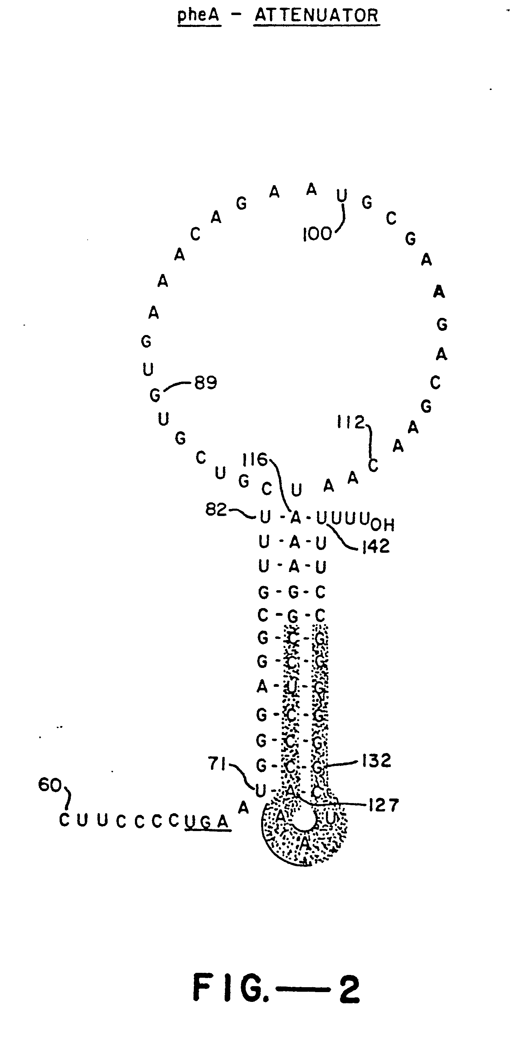

Another enzyme which may be overexpressed which is necessary for biosynthesis of the desired amino acid in the preferred embodiment (wherein phenylalanine is overexpressed) is the pheA gene. This may be accomplished by gene amplification by inserting DNA molecules containing pheA into the multicopy plasmid and transforming the E. coli host with the plasmid. However, in some cases, the entire intact pheA gene will not be inserted into the plasmid. That is, the gene may be modified in order to deregulate the attenuator, abolish the operator-repressor interaction, and/or to substitute a pheA gene encoding an enzyme resistant to feedback inhibition by phenylalanine. For example, an EcoRI-BamHI fragment containing the pheA gene (from the same origin as the aroF gene) may be first subcloned onto a multicopy plasmid, such as pBR322; then the attenuator can be partially deleted by excision of a specific 17-base pair DNA restriction fragment. The general approach for this excision is shown in FIGS. 2 through 4.

As a further modification, the operator-repressor interaction of pheA may be abolished by using as a host strain an E. coli which contains no active repressor. This may be accomplished by engineering an operator-constitutive mutation on the pheA operon contained in the plasmid, or by joining the pheA coding region and attenuator, without an operator, to the end of the aroF gene so that the expression of pheA is under control of the aroF gene promoter.

In a second aspect of the invention, a feedback resistant mutation is used so that expression of the desired amino acid is not affected by feedback inhibition. In the preferred embodiment, this is obtained by selecting for a feedback resistant mutation in the chromosomal or plasmid-containing pheA gene, utilizing resistance to certain phenylalanine analogs, such as m-fluorophenylalanine. If the mutation is on the chromosome, that pheA gene is then isolated and inserted onto a multicopy plasmid.

The third aspect of the present invention is to inactivate genes whose products lead to decreased yields of the desired amino acid by inhibiting or repressing expression of necessary enzymes. For example, such products may cause diversion of carbon flow to a secondary, undesired product or inhibit or repress expression of the enzymes which inhibit overproduction of the desired amino acid. In Figure 1, it may be seen that the presence of a functional tryA gene causes production of tyrosine which is undesirable, because not all of the prephenate made will be converted to phenylalanine, the desired amino acid in the preferred embodiment. Also excess tyrosine in the cell will repress aroF expression and inhibit its gene product. It is preferred that inactivation of the tyrA gene be performed by transposon mutagenesis on the genomic DNA of the host organism either directly or with a tyrA-containing plasmid intermediate. Production of tryptophan can be similarly abolished by inactivating the trp operon.

It is preferred that the aroF gene and pheA gene be inserted into one or more multicopy plasmids and transformed into the appropriate E. coli culture. Multicopy plasmids which are useful in bacteria are well known such as ColE1 and its derivatives, including pBR322, pBR328 and the like.

Site specific mutagenesis may be performed according to the procedures set forth by Wallace et al (1981), Nuc. Acids Res., 9, 3647 and Dalbadie-McFarland et al (1982), Proc. Natl. Acad. Sci., 79, 6409. Typically, the nucleotide sequence in the vicinity surrounding the site to be mutagenized (target site) is first identified in the conventional manner by DNA sequencing. Then, a synthetic oligonucleotide is prepared which is complementary to a sequence in the vicinity of the target site to be mutagenized, except that the oligonucleotide contains a deletion or nucleotide substitution(s). The oligonucleotide is hybridized to a single-stranded region of the DNA containing the portion of the gene to be mutagenized and the oligonucleotide extended by DNA polymerization to yield an intact, double-stranded, plasmid. By conventional amplification techniques, the double-stranded DNA will result in two forms of the gene, one which is mutagenized (see FIG. 4). The colonies containing the mutagenized gene may be selected by hybridization to the same oligonucleotide, which functions as a probe.

Transposon mutagenesis may generally be conducted by use of a lambda phage containing a transposon such as Tn5 or Tn10. For example, a plasmid containing the gene to be mutagenized in a host strain may be infected by lambda containing the transposon. The resultant colonies may be tested for insertion of the transposon into the target gene by drug resistance (a marker on the transposon) and restriction endonuclease mapping. The mutated gene may then be forced into the chromosome of the host cell by transformation of the host with a second plasmid which is incompatible with the first plasmid. Colonies containing the second plasmid and the transposon may then be found by screening for the second plasmid's marker and a nonfunctional target gene. These colonies will contain the mutated target gene in the genomic DNA. The transposon can also be inserted directly into the chromosomal DNA by infection of the host strain with transposon-containing lambda phage and selection of colonies that both contain the selection marker of the transposon and a nonfunctional target gene.

Conventional methods for identifying host cells containing the target gene(s) residing on a plasmid and/or within the genomic DNA will be utilized. In the case of screening for a vector, colonies are first identified by loss and retention of certain selection markers carried on the vector, such as resistance to antibiotics. Further characterization involves assaying the individual colonies for activity of the relevant enzyme(s) This includes the ability to distinguish feedback resistant vs inhibitable enzymes, repression vs derepression of an enzyme's expression, and loss of functionality. The proper fragments of DNA within a vector will be confirmed by conventional restriction endonuclease mapping and, when necessary, by conventional DNA sequencing analysis.

Conditions for culturing microorganisms such as bacteria are well known and may be determined by those of ordinary skill. Purification of DNA may be conducted by conventional procedures such as by gel electrophoresis, HPLC, and the like.

In accordance with the teachings of the present invention, desired amino acids may be expressed in unexpectedly high concentrations. Exemplary yields may be greater than 11.5 grams amino acid per liter of microorganisms in a broth initially containing about 50 g. glucose per liter.

Having described the preferred embodiments of the present invention, the following are presented by way of example and are not intended to limit the scope of the present invention.

## EXAMPLE 1

### Construction of plasmids phe14, tyr25 and phearo2

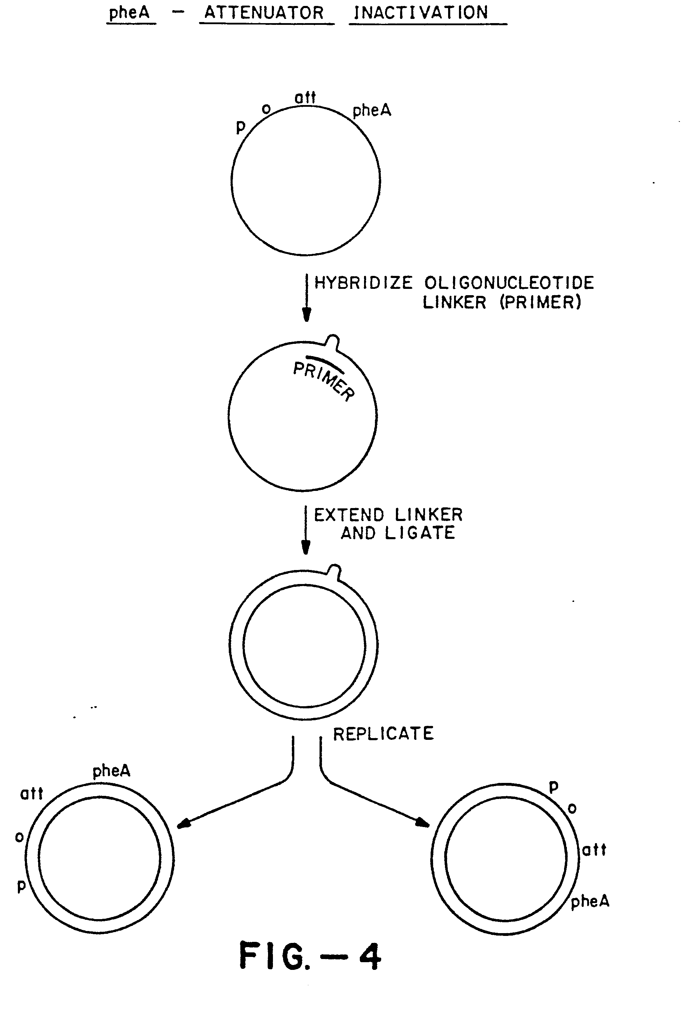

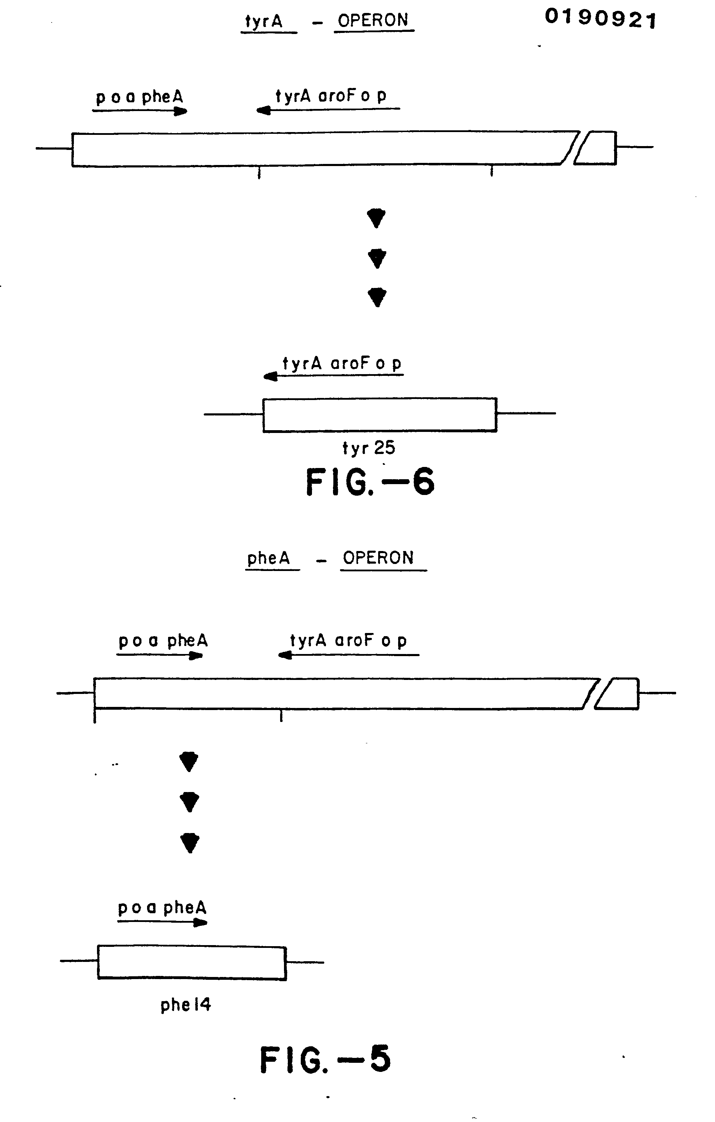

Plasmid phe14 was derived from plasmid pheT5 (Zurawski, et al, ibid); specifically, the 2.1-kb pheA-containing EcoRI-BamHI fragment from pheT5 was ligated into the vector pBR322 that had been restricted with endonucleases EcoRI and BamHI (see Figure 5). Plasmid tyr25 was also derived from plasmid pheT5; specifically, the 2.8-kb tyrA-containing BamHI fragment from pheT5 was ligated into the vector pBR322 that had been restricted with endonuclease BamHI (see Figure 6). Plasmid phearo2 was constructed by ligating the BamHI fragment containing the tyrA gene into the BamHI site of plasmid phe14 in the orientation depicted in Figure 7.

## EXAMPLE 2

### Partial deletion of the attenuator region of the phe gene

The restriction map of the promoter and leader region of the pheA gene were deduced from the nucleotide sequence disclosed by Zurawski, et al. (ibid). The proposed secondary structure of the attenuator region (bases 71 through 142) in its termination mode is shown in Figure 2. The bases 120 through 137 of the attenuator are to be deleted and a fragment containing the deletion used as an oligonucleotide to mutate the pheA operon.

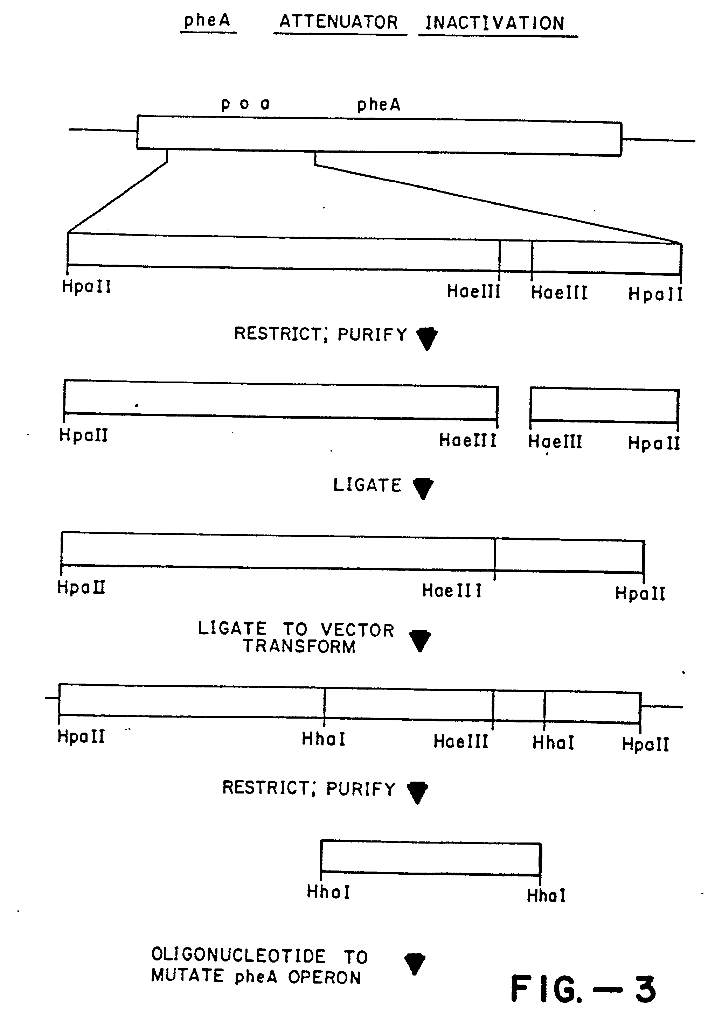

Referring to FIG. 3, firstly, the 630-bp fragment from the HpaII digestion of phe14 (see example 1) was prepared and isolated, the proper fragment being identified by size (migration through an acrylamide gel) and by restriction endonuclease mapping thereof. This fragment will be digested with HaeIII to produce three fragments: HpaII-HaeIII of 440 bp; HaeIII-HaeIII of 17 bp; and HaeIII-HpaII of 173 bp. The 440-bp and 173-bp fragments are purified and ligated together at their respective HaeIII ends to produce a HpaII-HpaII 613-bp fragment. The 613-bp fragment is then ligated into the ClaI site of pBR322; the 613-bp fragment is directly ligatable into the ClaI 5' extended ends since the respective extended ends of HpaII and ClaI are complementary.

A 227-bp fragment containing the mutated attenuator region is subsequently excised from the vector with HhaI. The 227-bp fragment is then used as an oligonucleotide primer to mutate the pheA gene in any appropriate vector containing the gene (e.g., phe14, phearo2), as illustrated in FIG. 4.

## EXAMPLE 3

### Fusion of the pheA coding region to the aroF gene

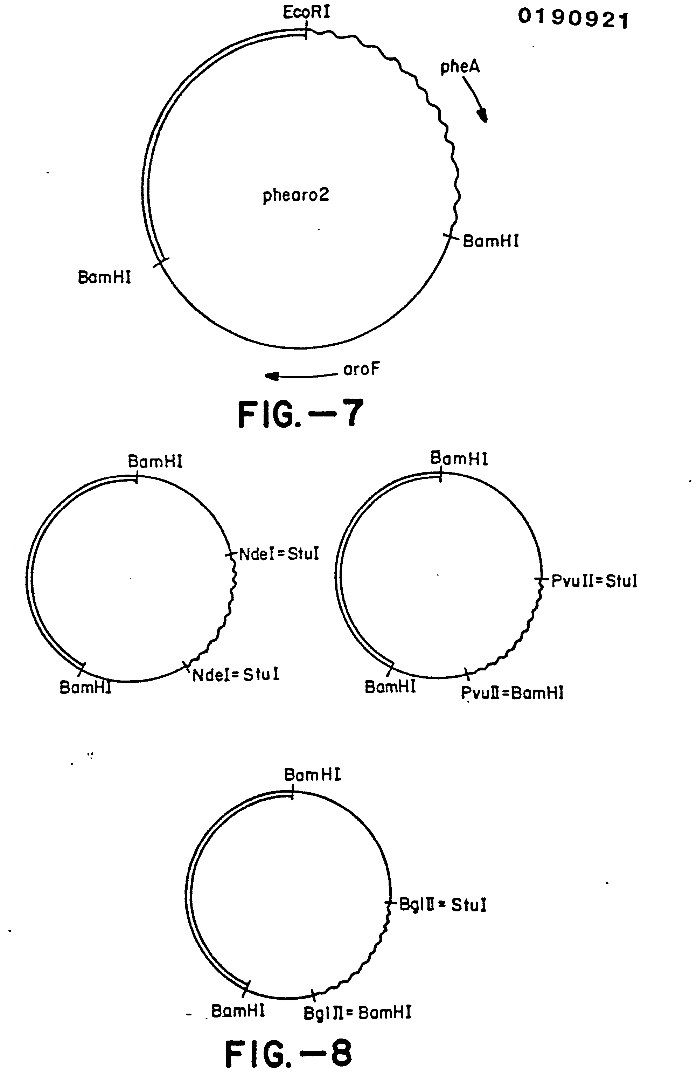

As an alternative to inactivating the attenuator and operator of the pheA gene, the coding region of the pheA gene (i.e., lacking the promoter, operator, and the leader region [that portion of the gene which is transcribed but not translated]) may be fused to the aroF gene such that expression of the pheA gene is under control of the aroF promoter. The leader region of the pheA gene is well characterized (see Zurawski, et al., ibid). Thus, restriction with StuI cuts the gene downstream from the attenuator region but upstream of the structural coding region. Referring to FIG. 8, as a starting material, the 1.4 kb StuI-BamHI fragment containing the pheA coding region is purified from plasmid phel4. Plasmid tyr25, containing the aroF gene is then restricted with either NdeI (within the aroF coding region), PvuII (close to the carboxy terminus of the coding region), or BglII (downstream from the coding region). The StuI-BamHI pheA containing fragment is ligated into each of these vectors, using linkers when necessary. Each construction will result in expression of both the pheA and aroF genes from the aroF promoter, and thus will be an operon regulated by the amount of tyrosine in the cell. It is noted that the construction where the NdeI site of aroF is fused to the StuI site of pheA does not result in a functional aroF gene.

## EXAMPLE 4

### Inactivation of the tyrA operon in the host genome

In order for the plasmid described above to achieve the desired result, the host cell must be auxotrophic for tyrosine. Otherwise some portion of the chorismate produced will be diverted to tyrosine rather than phenylalanine production. Thus, the genomic tryA gene must be inactivated. This may be done by transposon mutagenesis using a lambda phage containing Tn5 (lambda b221c1857::Tn5, from D. Berg). E. coli strains W3110trpRtnaA are infected with lambda::Tn5 and selected for resistance to kanamycin (transposon Tn5 marker) and tyrosine auxotrophy. The selected strains are then transformed with tyr25 (which contains a functional tyrA gene). If complementation occurs, the mutation causing auxotrophy is in the tyrA gene, as desired. To verify that transposon Tn5 is inserted into the tyrA gene, chromosomal DNA from strains showing complementation are restricted with endonuclease EcoRI or BamHI and the resultant digests run on a gel and the DNA blotted onto nitrocellulose. The DNA patterns are probed with DNA fragments corresponding to regions of the tyrA gene and of transposon Tn5. Digests showing that the two probes hybridized to the same site on the genome confirm that transposon Tn5 is inserted to the chromosomal tyrA gene.

The properly identified strain, e.g., W311trpRtnaA-tyrA::Tn5 (also called RTC9) is then transformed with the vector described above to construct a strain that overproduces phenylalanine.


EXAMPLE 5


Fermentation of phearo2 in RTC9


Plasmid phearo2 is transformed into the host RTC9. The ability of the resultant production strain to make phenylalanine was tested by growing it in a 500-ml Erlenmeyer flask containing 100 ml defined medium. The medium consists of (per liter) 50 g glucose, 0.5 g potassium phosphate, 5.8 g ammonium chloride, 10 mg ferric chloride, 0.8 g magnesium chloride, 0.36 g potassium sulfate, 80 mg tyrosine, 1.5 g sodium citrate, 4% calcium carbonate, and 0.16 ml Trace Elements solution. (Trace elements solution is 3 uM NH4MoO7, 400 uM H3BO3, 10 uM CuSO4, 80 uM MnCl2, 10 uM ZnSO4.) Fermentation times of 48 to 60 hours at 37 C typically result in yields of 9 to 10 g/l phenylalanine.

-17-

## EXAMPLE 6

### Construction of a plasmid containing an aroF-feedback resistant gene product

Directed in vitro mutagenesis of the DNA fragment containing the aroF gene is carried out utilizing either nitrous acid (in a similar manner to Warburton, et al, (1983) Nucl. Acids Res. 11, 5837) or misincorporation of a specific nucleotide at random points along the fragment in a manner similar to that described by Abarzua and Marians (1984) Proc. Natl. Acad. Sci. 81, 2030. In the latter procedure, a plasmid containing aroF is nicked with either deoxyribonuclease I or restriction endonuclease HpaII, followed by limited exposure for various lengths of time to exonuclease III. A thio-containing nucleotide is then misincorporated into the DNA, followed by repair with DNA polymerase I and ligation with T4 DNA ligase. Colonies containing a feedback resistant aroF gene product are then isolated due to their ability to grow on 3-fluorotyrosine, a tyrosine analogue. That the mutation indeed results in feedback resistance to tyrosine is verified by an enzymatic assay for DAHP symthetase; in this case the aroF encoded enzyme should no longer be inhibited by tyrosine; normally, the wild type allele encodes an enzyme whose activity is inhibited 93% by 1 mM tyrosine.

## EXAMPLE 7

### Construction of a plasmid containing an operator-constitutive aroF gene

The procedure for obtaining an operator constitutive mutation is similar to that described in Example 6, except that the DNA fragment containing the operator and promoter is the one exposed to mutagenesis. The screening technique is also similar, i.e., ability to grow on 3-fluorotyrosine; however, the results of the enzymatic assay will differ: In this case, a larger than normal quantity of DAHP synthetase encoded by aroF will be made regardless of whether there is tyrosine in the medium, but 1 mM tyrosine will still inhibit the aroF gene product.

It will be appreciated that in general the invention includes a microorganism capable of overexpressing an amino acid wherein the chromosomal DNA of said microorganism has been altered and/or the microorganism has been tranformed with at least one expression vector wherein said alterations or vector causes

(a) overexpression of at least one enzyme necessary for the biosynthesis of said amino acid;

(b) elimination of feedback inhibition of expression of said amino acid by mutation of at least one gene necessary for the biosynthesis of said amino acid; and

(c) inactivation of at least one gene which produces a product which inhibits or represses expression of said enzyme in step (a).

Such a microorganism can be further defined by any of the features hereinbefore described.

When the method described is put into practice by transforming a microorganism, the invention further includes vectors capable of transforming such microorganisms.

0190921

CLAIMS

1. A method for overexpressing an amino acid in a microorganism comprising the step of altering the chromosomal DNA of said microorganism and/or transforming said microorganism with at least one expression vector wherein said alterations or vector causes

(a) overexpression of at least one enzyme necessary for the biosynthesis of said amino acid;

(b) elimination of feedback inhibition of expression of said amino acid by mutation of at least one gene necessary for the biosynthesis of said amino acid; and

(c) inactivation of at least one gene which produces a product which inhibits or represses expression of said enzyme in step (a).

2. A method according to claim 1 wherein said microorganism comprises bacteria.

3. A method according to claim 1 or claim 2 wherein said bacteria are selected from the group consisting of _Escherichia_, _Bacillus_, _Corynebacteria_ and _Brevibacteria_.

4. A method according to claim 3 wherein said bacteria comprise _Escherichia coli_.

5. A method according to claim 4 wherein said overexpression is performed by subcloning the _aro_F operon or the _phe_A onto a multicopy plasmid and transforming said plasmid into _E. coli_.

0190921

6. A method according to claim 4 wherein said elimination of feedback inhibition is performed by a mutant pheA gene and/or a mutant aroF gene.

7. A method according to claim 4 wherein said overexpression is performed by excising the attenuator region and performing site-directed mutagenesis on the pheA operon to overproduce phenylalanine.

8. A method according to claim 5 wherein the pheA gene is subcloned onto the muticopy plasmid containing the aroF gene.

9. A method according to claim 4 wherein said inactivation is performed by mutagenizing E. coli host chromosomal DNA by transposon mutagenesis to inactivate the tyrA gene.

10. A method according to any one of claims 1 to 9 wherein said amino acid comprises phenylalanine.

0190921

FIG.—I

0190921

## pheA - ATTENUATOR

FIG.—2

FIG. — 3

pheA — ATTENUATOR INACTIVATION

FIG. — 4

tyrA — OPERON

0190921

p o a pheA    tyrA aroF o p

tyrA aroF o p

tyr 25

# FIG.—6

pheA — OPERON

p o a pheA    tyrA aroF o p

p o a pheA

phe 14

# FIG.—5

FIG. —7

FIG. —8

0190921